(19) Europäisches Patentamt
European Patent Office
Office européen des brevets

(11) Veröffentlichungsnummer: **0 072 994**
**B1**

(12) **EUROPÄISCHE PATENTSCHRIFT**

(45) Veröffentlichungstag der Patentschrift: **11.06.86**

(51) Int. Cl.⁴: **A 45 D 20/00**, A 45 D 20/40, A 61 N 5/06

(21) Anmeldenummer: **82107434.1**

(22) Anmeldetag: **16.08.82**

(54) **Wärmebehandlungsgerät für Kopfhaare.**

(30) Priorität: **27.08.81 DE 3133851**

(43) Veröffentlichungstag der Anmeldung:
**02.03.83 Patentblatt 83/9**

(45) Bekanntmachung des Hinweises auf die Patenterteilung:
**11.06.86 Patentblatt 86/24**

(84) Benannte Vertragsstaaten:
**AT BE DE FR IT NL SE**

(56) Entgegenhaltungen:
**CH - A - 604 606**
**DE - U - 7 810 814**
**FR - A - 944 623**
**FR - A - 2 314 638**
**GB - A - 611 663**
**GB - A - 617 081**

(73) Patentinhaber: **Wella Aktiengesellschaft, Berliner Allee 65, D-6100 Darmstadt (DE)**

(72) Erfinder: **Hollmann, Siegfried, Am Forsthaus Gravenbruch 67, S-6078 Neu-Isenburg (DE)**
Erfinder: **Hoch, Dieter, Ringstrasse 48, D-6102 Pfungstadt (DE)**
Erfinder: **Bollinger, Heribert, Bruneckerstrasse 5, D-6080 Gross-Gerau (DE)**

**EP 0 072 994 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft ein Wärmebehandlungsgerät zum Erwärmen der menschlichen Kopfhaare mit mehreren an einem Tragearm befestigten Infrarotstrahlern.

Es sind bereits verschiedene Wärmebehandlungsgeräte bekannt, beispielsweise wie in den DE-U-78 10 814 und GB-A-611 663 beschrieben, bei der mehrere freibewegliche Tragarme mit je einem Infrarotstrahler mit zylinderförmigem Strahlenkegel an einem Bodenstativ vorgesehen sind. Geräte dieser Art haben den Nachteil, daß sie keine gleichmäßige Bestrahlung der gesamten Kopfoberfläche erzielen und die Tragarme durch Probieren der Lageeinstellung auf die zu behandelnde Kopfoberfläche eingestellt werden müssen, um örtliche Überhitzungen des Haares zu vermeiden. Darüberhinaus wird das Haar mit einer zu geringer Gesamtheizleistung bestrahlt, wodurch eine lange Einwirk- bzw. Behandlungszeit erforderlich ist.

Die Erfindung hat sich zur Aufgabe gestellt, eine neuartige Bauweise eines Wärmebehandlungsgerätes der eingangs genannten Art, insbesondere zur Dauerwell-, Kur- bzw. Trocknungsbehandlung des menschlichen Kopfhaares im Friseursalon anzugeben, wodurch seine Bedienung einfacher und das Risiko einer örtlichen Überhitzung von Haarpartien verringert wird.

Diese Aufgabe wird gemäß der Erfindung dadurch gelöst, daß die Infrarotstrahler im wesentlichen als Kreissegment gekrümmte linienförmige Heizkörper ausgebildet sind und jeweils einen außenkreisseitigen im wesentlichen koaxial zum Heiskörper (23) gekrümmten metallischen Reflektor aufweisen, daß zwei seitliche Infrarotstrahler und wenigstens ein in Gebrauchslage oberhalb angeordneter mittlerer Infrarotstrahler mit mindestens einem an einem Bodenstativ bzw. an einem Wandarm beweglich gelagerten Tragearm fest verbunden sind, daß die in Richtung eines Krümmungsradius jeweils verlaufenden Mittelachsen zweier seitlich angeordneter Infrarotstrahler miteinander einen Winkel von wenigstens 180° einschließen und daß das Verhältnis aus Länge des gekrümmten linienförmigen Heizkörpers zu dessen Krümmungsradius wenigstens 0,2 beträgt.

Vorteil der offenen Bauweise ist, daß man während der Strahlungseinwirkung am Haar arbeiten kann. Vorteilhaft vor allem beim Trocknen von dauergewelltem Haar. Es ist zweckmäßig, wenn im Haar, vorzugsweise auf der Oberfläche eines mit einer Haarsträhne bewickelten Lockenwicklers, ein Temperaturfühler angeordnet ist, der die Heizleistung der Infrarotstrahler mittels einer vorzugsweise im Sedienpult angeordneten Regelbausteins auf eine vorgegebene Solltemperatur einregelt, wobei durch die feste geometrische Zuordnung von dem zu behandelnden Haar und den Infrarotstrahlern reproduzierbare Haarbehandlungsergebnisse erzielt werden.

Bei der Wärmebehandlung, ganz besonders bei der sehr empfindlichen Dauerwellbehandlung des Haares, hängt das fachliche Ergebnis in relativ engen Grenzen von der Wärmeenergie und der Einwirkzeit ab. Mit Hilfe der Temperaturregelung, gesteuert über den Temperaturfühler, läßt sich die Einflußgröße Wärmeenergie sehr genau dosieren. Vorteilhaft ist beim erfindungsgemäßen Wärmebehandlungsgerät die sehr gleichmäßige Bestrahlung aller Haarpartien (Nacken, Oberkopf, Seiten) mit hoher Strahlungsenergie.

Um chemische Präparate schneller und optimaler zu verarbeiten ist eine genau dosierte Wärmezufuhr von großem Nutzen. Bei Einwirkung dieser Präparate ohne zusätzliche Wärmezufuhr ist allein die augenblickliche Raumtemperatur als Energiespender vorhanden. Da diese Raumtemperatur stark schwankt (Sommer, Winter, Tageszeit abhängig) ist eine exakte Angabe von Einwirkzeiten unmöglich. Mit Hilfe eines Wärmebehandlungsgerätes soll auf eine bestimmte Haartemperatur geregelt werden, die oberhalb der Raumtemperatur liegt. Um eine gleichmäßige Reaktion des chemischen Präparates zu erreichen, muß die Temperatur an jeder Stelle des Haares möglichst konstant gehalten werden.

Ausgehend von der Beschaffenheit der chemischen Produkte wurde weiterhin untersucht, welche Art von Wärmeübertragung sich am besten eignet. Da ein sehr hoher Prozentsatz an Wasser sich in den Produkten befindet, wurde eine Strahlungswärmequelle ausgewählt, die ihr Strahlungsmaximum in dem Bereich hat, in dem das höchste Absorptionsmaximum von Wasser liegt. Es zeigt sich, daß der optimale Sereich zwischen 2,3µm bis 2,7 µm liegt. Diese Ergebnisse decken sich eindeutig mit der Theorie der Absorption von dünnen Wasserschichten.

Weitere Fortbildungen und Ausgestaltungen der Erfindung sind in den Unteransprüchen gekennzeichnet und werden nachstehend in Verbindung mit den zwei Ausführungsbeispiele darstellenden, teilweise schematisch vereinfachten Figuren beschrieben. In diesen sind einander entsprechende Teile mit gleichen Bezugszeichen versehen, und es sind alle zum Verständnis der Erfindung nicht notwendigen Einzelheiten fortgelassen worden.

Fig. 1 zeigt eine Seitenansicht einer erfindungsgemäßen Ausführungsform als Bodengerät in einer ersten Gebrauchsstellung mit in eine obere Kippstellung geschwenkten seitlichen Infrarotstrahlern in Verbindung mit einem zu behandelnden Kopf.

Fig. 2 zeigt einer Rückenansicht in Richtung des Pfeiles X der in Fig. 1 gezeigten Ausführungsform, ebenfalls in erster Gebrauchsstellung.

Fig. 3 zeigt eine Vorderansicht der in Fig. 1 gezeigten Ausführungsform, ebenfalls in erster Gebrauchsstellung.

Fig. 4 zeigt eine Vorderansicht der in Fig. 1 gezeigten Ausführungsform ähnlich Fig. 3, jedoch in Ruhestellung mit aus der ersten Gebrauchsstellung in die Mitte eingeklappten seitlichen Infrarotstrahlern.

Fig. 5 zeigt einen Horizontalschnitt entlang der Linie A-A der in Fig. 1 gezeigten Ausführungsform in erster Gebrauchsstellung in Verbindung mit einem zu behandelnden Kopf.

Fig. 6 zeigt ein Vertikalschnitt entlang der Linie B-B in Fig. 3 durch die mittleren Infrarotstrahler der in Fig. 1 gezeigten Ausführungsform in Verbindung mit einem zu behandelnden Kopf.

Fig. 7 zeigt einen Vertikalschnitt entlang der Linie C-C durch einen seitlichen Infrarotstrahler senkrecht zu dem in Fig. 5 gezeigten Horizontalschnitt der ersten Ausführungsform in erster Gebrauchslage in Verbindung mit einem zu behandelnden Kopf.

Fig. 8 zeigt einen Vertikalschnitt ähnlich Fig. 7 - jedoch in einer zweiten Gebrauchsstellung, bei der der seitliche Infrarotstrahler abweichend in eine untere Kipplage für langes Kopfhaar geschwenkt ist.

Fig. 9 zeigt eine rückwärtige Ansicht des zu behandelnden Kopfes mit aufgewickelten Lockenwicklern, wobei das von einer optischen Einstellvorrichtung auf dem Kopf erzeugte Fadenkreuz sowohl bei richtigem als auch falschem Kopfabstand dargestellt ist.

Fig. 10 zeigt eine vergrößerte Ansicht auf das Bedienungsfeld des in Fig. 2 bzw. Fig. 5 gezeigten Bedienungspultes.

Fig. 11 zeigt eine Seitenansicht einer zweiten Ausführungsform in erster Gebrauchsstellung ähnlich Fig. 1, jedoch abweichend als Wandgerät ausgebildet.

Das in Fig. 1 bis 3 dargestellte Wärmebehandlungsgerät besteht aus einem Rollen 1 tragenden Stativkreuz 2, das ein vertikales, wahlweise längsverschiebbares und axial verdrehbares Standrohr 3 trägt. Am oberen Ende 4 dieses Standrohres 3 sind am zwei Seitenarmen 5 jeweils ein seitlicher Infrarotstrahler 6 angeordnet. Am oberen Ende 4 ist weiterhin ein im wesentlichen horizontal vorragender Tragearm befestigt, an dem zwei im wesentlichen nach unten strahlende mittlere Infrarotstrahler 9 befestigt sind. Desweiteren ist an oberen Ende 4 des Standrohres 3 ein Bedienpult mit einem Bedienfeld 11 angeordnet, der auf der von den Infrarotstrahlern 6 und 9 abgewendeten Seite zuliegen kommt. Ein daran befestigter Griff 10a erlaubt wahlweise eine gemeinsame Höhenverstellung bzw. Drehung der Infrarotstrahler 6 und 9.

Mit diesem Bedienpult 10 steht ein Temperaturfühler 12 über eine flexible Leitung 13 in Verbindung. In Fig. 1 ist ein menschlicher Kopf 14 mit von Lockenwicklern 15 aufgewickeltem Haar im Strahlungsbereich der Infrarotstrahler 6 und 9 angedeutet, aus der die Behandlungsanordnung ersichtlich ist. Von einem der Lockenwickler 15 wird der Temperaturfühler 12 mittels einer nicht dargestellten Klammer in Kontakt mit der darauf aufgewickelten Haarsträhne gehalten.

Zur Einstellung des optimalen Kopfabstandes zwischen den Infrarotstrahlern 6 und 9 und dem Kopf 14 ist am oberen Ende 4 des Standrohres 3 eine optische Einstellvorrichtung zur Lagefixierung des Kopfes 14 angeordnet, die aus zwei nebeneinander angeordneten Projektionslampen 17 gebildet wird, deren optische Strahlachse 18 mit einem Winkel α von vorzugsweise 24° bis 35 bilden. Jede dieser beiden Projektionslampen 17 erzeugen auf dem zu behandelnden Kopf 14 im Bereich des Haaransatzes (Nackenkontur 27) einen waagrechten hellen Strich 20 bzw. senkrechten Strich 21, der bei richtig vorgegebenem Kopfabstand jeweils scharf abgebildet werden und vorzugsweise, wie in Fig. 9 dargestellt, ein gleichschenkliges Kreuz 22 bilden. Bei falschem Kopfabstand liegen diese beiden Striche 20 bzw. 21 nebeneinander, wie zusätzlich in Fig. 9 gestrichelt angedeutet.

Die Seitenarme 5 sind mittels im wesentlichen um eine senkrechte Achse schwenkbares Gelenk 19 jeweils mit dem oberen Ende 4 des Standrohres 3 verbunden, so daß in der Ruhestellung die Seitenarme 5 mit den seitlichen Infrarotstrahlern 6 in die Mitte aufeinander zu geklappt sind, wie in Fig. 4 gezeigt. Hierdurch benötigt das Wärmebehandlungsgerät einen geringeren Platzbedarfbei Nichtbenutzung.

Aus den Figuren 3 und 5 ist der Aufbau der Infrarotstrahler 6 und 9 ersichtlich. Jeder Infrarotstrahler 6 bzw. 9 weist einen länglichen, leicht gebogenen Rohrheizkörper 23 aus Quarzglas auf, der von einem Reflektor 24 auf dessen in Gebrauchslage von dem Kopf abgewendeten Seite angeordnet ist. Die offene Seite jedes Infrarotstrahlers 6 bzw. 9 wird von einem als Berührschutz wirkendem Gitter 25 abgedeckt.

Aus der Fig. 5 ist die Längskrümmung des Rohrheizkörpers 28 ersichtlich, der eine Krümmungsradius R von vorzugweise 800 mm aufweist. Die Mittelachse 26 jedes der beiden seitlichen Infrarotstrahler 6 bildet die jeweilige Hauptstranlrichtung. Die Mittelachsen 26 beider seitlicher Infrarotstrahler 6 schließen zwischen sich einen Winkel β von vorzugsweise 108° ein, wobei der Abstand S des gemeinsamen Schnittpunktes M vom jeweiligen Rohrheizkörper 23 ca. den halben Krümmungsradius R beträgt.

Das Verhältnis aus Länge L jedes gekrümmten Rohrheizkörpers 23 zu seinem jeweiligen Krümmungsradius R beträgt worzugsweise wenigstens 0,3 besonders beworzugt wird ein Verhältnis von 0,37 bis 0,57 Es hat sich gezeigt, daß es gunstig ist, wenn die aufgenommene Leistung eines Infrarotstrahlers 250 W beträgt und der Mindestabstand D zwischen jedem Rohrheizkörper 23 und dem Haar -bzw. bei Verwendung von Lockenwicklern- von jedem Lockenwickler vorzugsweise 25 % bis 30 % des jeweiligen Krümmungsradius R beträgt. Die Längsachse jedes seitlichen Infrarotstrahlers 6

weist einen Erhebungswinkel φ von etwa 12° in Sezug auf die Horizontale 29 auf wie in Fig. 1 angedeutet.

In Fig. 5 sind zusätzlich die optischen Strahlachsen 18 der Projektionslampen 17 eingezeichnet, deren gemeinsamer Schnittpunkt auf der gestrichelt angedeutenden Nackenkontur 27 im Bereich des Haaransatzes zu liegen kommt.

Die in Fig. 6 gezeigte Geometrie der mittleren Infrarotstrahler 9 ist analog der in Fig. 5 gezeigten Anordnung der seitlichen Infrarotstrahler 6. Die Mittelachsen 28 beider mittlerer Infrarotstrahler 9 schließen zwischen sich einen Winkel γ von vorzugsweise 68,5° ein, wobei der Abstand H des gemeinsamen schnittpunktes P vom jeweiligen Rohrheizkörper 23 ebenfalls etwa den halben Krümmungsradius R beträgt. Weiterhin schließt die Mittelachse 28 des unteren der beiden mittleren Infrarotstrahlern 9 mit der Horizontalen 29 einen Winkel ψ von etwa 55 ein. Hierdurch wird erreicht, daß eine bequeme Kopfhaltung im Sitzen eingenommen werden kann.

Der Mindestabstand D jedes Rohrheizkörpers 23 vom Haar -bzw. bei Verwendung von Lockenwicklern- vom jeweiligen Lockenwickler beträgt vorzugsweise 25 bis 30 % des jeweiligen Krümmungsradius R. Der Reflektor 24 in jedem mittleren Infrarotstrahler 9 ist im Querschnitt vorzugsweise trapezförmig mit gleich langen Schenkeln ausgebildet, dessen Grundseite senkrecht zur optischen Strahlachse 18 angeordnet ist. Hierdurch wird erreicht, daß die Strahlung nicht längs einer Linie auf der Kopfoberfläche fokussiert wird, sondern eine möglichst gleichmäßige Bestrahlung der Kopfoberfläche erreicht wird. Wie aus Fig. 7 besser ersichtlich beträgt der halbe Öffnungswinkel δ des trapezförmigen Reflektors 24 etwa 30°. In Bezug auf die Horizontale 29 ist die Mittelachse 26 der seitlichen Infrarotstrahler 6 um ein Winkel von vorzugsweise 10° nach oben in eine erste Gebrauchslage für vorzugsweise auf Lockenwickler aufgewickeltes Haar bzw. kurzes Haar geneigt. Zur Verwendung von langem, insbesondere schulterlangem Haar ist der seitliche Infrarotstrahler 6 in eine zweite Gebrauchslage nach unten umschwenkbar, wie in Fig. 8 gezeigt. In diesem Fall ist die Mittelachse 26 um einen Winkel λ von vorzugsweise 20° in Bezug auf die Horizontale 29 nach unten geneigt. Der schwenkbereich (ε + λ) der beiden seitlichen Infrarotstrahler 6 beträgt somit vorzugsweise 30°.

Mit dem erfindungsgemäßen Wärmebehandlungsgerät sind drei unterschiedliche Behandlungsverfahren der Kopfhaare möglich, und zwar ein sogenanntes Dauerwellprogramm, ein sogenanntes Kur- und Färbeprogramm und ein Trocknungsprogramm, die nachfolgend einzeln beschrieben werden.

**a) Dauerwellprogramm**

Das in Fig. 10 dargestellte Bedienfeld 11 weist rechts unten sechs Drucktasten eines ersten Tastenfeldes 30 zur Eingabe der Zahlen 1 bis 6 auf, wobei jeweils jede Drucktaste einen separaten Zahlenwert zugeordnet ist und auf ihrer Bedienungsseite mit dem entsprechenden Zahlenwert beschriftet ist. Es können nacheinander fünf unterschiedliche Zahlenwerte durch Drücken der jeweiligen Drucktaste eingegeben werden, wobei der jeweils nacheinander eingegebene Zahlenwert in fünf einstelligen 7-segmentanzeigen (LED) 31 untereinander und von oben nach unten in einer entsprechend zugordneten Anordnung angezeigt werden. Beispielsweise sind auf den Sedienfeld 11 in Fig. 10 nacheinander die Zahlenwert 1-1-1-1-5 eingegeben worden und entsprechend auf den einstelligen 7-segmentanzeigen 31 angezeigt. Bei Eingabe eines unzulässigen Zahlenwertes blinkt die entsprechende einstellige 7-segmentanzeige 31 und das gewählte Dauerwellprogramm wird gesperrt. Der in der untersten einstelligen 7-segmentanzeige 31 dargestellte Zahlenwert (in Fig. 10 mit "Ziffer 5" versehen) ist eine Codenummer für die Art der am Haar vorher durchgeführten Dauerwellbehandlung. Die im internen Programmspeicher hierzu gespeicherte Einwirkzeit wird anschließend an einer zweistelligen 7-segmentanzeige 82 angezeigt (in Fig. 10 ist beispielsweise eine Einwirkzeit von 15 min. dargestellt). Nach Drücken der mit "D" bezeichneten Drucktaste eines zweiten Tastenfeldes 33 wird das Dauerwellprogramm gestartet. Hierbei wird die jeweils noch verbleibende Einwirkzeit an der zweistelligen 7-segmentanzeige 32 angezeigt und die beispielsweise dargestellte Einwirkzeit von 15 min. würde dann jeweils um ein Minute durch einen Rückwärtszähler erniedrigt. Das Dauerwellprogramm schaltet sich bei Erreichen der angezeigten noch verbleibenden Einwirkzeit von 0 min. ab. Während der Laufzeit dieses Dauerwellprogrammes heizen die seitlichen und mittleren Infrarotstrahler 6 und 9 das zu behandelnde Haar auf, wobei der von einem Lockenwickler 15 befestigte Temperaturfühler 12 die auf dem Haar erzeugte Temperatur mißt und durch eine interne Regelung die Heizleistung der Rohrheizkörper 23, vorzugsweise durch eine Ein- und Ausschaltung, geregelt wird. Es wird am Temperaturfühler 12 auf eine Solltemperatur im Bereich von 45° C bis 55° C, vorzugsweise 50° C, eingeregelt.

Nach Ablauf des Dauerwellprogrammes kann durch Drücken der mit "N" bezeichneten Drucktaste 34 eine vom Programm vorgebene Nacheinwirkzeit gewählt werden, die in der zweistelligen 7-Segmentanzeige 32 angezeigt wird. Durch erneutes Drücken der mit "D" bezeichneten Drucktaste des zweiten Tastenfeldes 33 wird der zugehörige Rückwärtszähler gestartet und die verbleibende Nacheinwirkzeit bis auf 0 entsprechend zurückgezählt. Dieser Vorgang ist mehrfach wiederholbar, solange bis die Dauerwellbehandlung abgeschlossen ist. Damit der Benutzer rechtzeitig das Ende der jeweiligen Heizdauer erkennen kann, beginnen bei einer in der zweistelligen 7-segmentanzeige 32 angezeigte Restlaufzeit von 3 min. deren Zahl zu blinken und ab einer Restlaufzeit von 10 sec. wird zusätzlich ein akustischer Summer gestartet. Zur Kontrolle der ursprünglich vorgegebenen Einwirkzeit bzw. Nacheinwirkzeit kann durch kurzzeitiges Drücken der mit "M" bezeichneten Drucktaste 35 deren Zahlenwert auf der zweistelligen 7-Segmentanzeige 32 kurzzeitig angezeigt werden. Weiterhin ist auf dem Bedienfeld 11 eine mit "C" bezeichnete Drucktaste 36 vorgesehen, bei deren Drücken das jeweilige Dauerwellprogramm angehalten wird und die löschbaren Speicherinhalte gelöscht werden und somit auch die Heizung der Infrarotstrahler 6 und 9 abgeschaltet werden. Beispielsweise kann bei Eingabe eines falschen Zahlenwertes mittels einer Drucktaste des ersten Tastenfeldes 30 durch Drücken der mit "C" bezeichneten Drucktaste 36 alle einstelligen 7-segmentanzeigen 31 gelöscht werden und danach erneut richtig eingegeben werden. Auf nicht näher dargestellten Beschriftungsfeldern 27, die neben den jeweiligen einstelligen 7-segmentanzeigen 31 angeordnet sind, sind die jeweils zugeordneten vorwählbaren Zahlenwerte für das Dauerwellprogramm angegeben.

**b) Kur- und Färbeprogramm**

Durch Wärmeeinwirkung soll hierbei der Einwirkprozeß auf das Haar beim Haarfärben bzw. beim Auftragen von Pflegeemulsionen verbessert, insbesondere beschleunigt werden. Mittels eines dritten Tastenfeldes 89 wird hierzu die Einwirkzeit durch Drücken seiner 10-er und 1-er Taste (entsprechende Bezeichnung 10 und 1 ist auf der Drucktaste 39 in Fig. 10 angebracht) eingestellt und von der zweistelligen 7-segmentanzeige 32 angezeigt. Solange die entsprechende Drucktaste gedrückt bleibt wird mit einem langsamen Takt die jeweilige 10-er-bzw. 1-er- stelle des Zahlenwertes der Einwirkzeit hochgezählt. Nach Drücken einer mit "F" gekennzeichneten Drucktaste des zweiten Tastenfeldes 33 wird das Kur- und Färbeprogramm gestartet und bei Erreichen einer restlichen Einwirkzeit von 0 min. durch Zeitablauf ausgeschaltet. Die solltemperatur am Haarfühler 12 liegt hierbei bei vorzugsweise 40°C, die bei einer Heizleistung von 250 W pro Infrarotstrahler 6 bzw. 9 mittels eines Impuls-Pausenverhältnisses von einer Einschaltdauer von 13 sec. zu einer Ausschaltzeit von 8 sec. vorzugsweise gebildet wird. Das Anzeigen des Programmendes dieses Programmes erfolgt analog wie beim Dauerwellprogramm.

**c) Trocknungsprogramm**

Zur Trocknung von feuchtem Haar kann das Wärmebehandlungsgerät ebenfalls benutzt werden. Die Einwirkzeit wird entsprechend mit dem dritten Tastenfeld 39 eingegeben und über eine mit "T" gekennzeichnete Drucktaste des zweiten Tastenfeldes 33 gestartet. Nach einem intern gespeichertem Programm werden die Infrarotstrahler mit maximaler Heizleistung zuerst vorzugsweise für 5 min. betrieben. Danach wird die Heizleistung mittels eines Impuls-Pausenverhältnisses von etwa 15 sec. Einschaltzeit zu 7 sec. Ausschaltzeit entsprechend herabgesetzt. Solche Impuls-Pausensteuerung erfolgt in bekannter Weise mittels sogenannter Triac-Halbleiter. Die behandelte Person kann mittels eines nicht dargestellten, an der Vorderseite des oberen mittleren Infrarotstrahlers angeordneten Schalters auf eine reduzierte Heizleistung mit einem Impuls-Pausenverhältnis von vorzugsweise 10 sec. Einschaltdauer zu 9 sec. Ausschaltdauer umschalten, wodurch die Temperatur des Haares entsprechend reduziert wird. Über einer mit "To" bezeichneten Drucktaste 40 können die seitlichen Infrarotstrahler 6 ausgeschaltet werden, so daß nur die mittleren Infrarotstrahler 9 beheizt werden. Alternativ kann durch Drücken der mit "Tu" bezeichneten Drucktaste 41 die mittleren Infrarotstrahler 9 ausgeschaltet werden, so daß nur die seitlichen Infrarotstrahler 6 beheizt werden. Dieses Zu- bzw. Abschalten kann bei allen drei Programmen vorgenommen werden und erlaubt eine individuelle Anpassung an die Frisur der zu behandelnden Person.

**Funktionsablauf:**

Eine Person, deren Haar zu behandeln ist, setzt sich beispielsweise auf einen entsprechenden Stuhl, der innerhalb des strahlungsbereiches der Infrarotstrahler angeordnet ist und nimmt eine Stellung ähnlich der in Fig. 1 gezeigten ein. Mittels der Projektionslampen 17 wird nun der erforderliche Mindestabstand D derart eingerichtet, daß der waagrechte Strich 20 und der senkrechte Strich 21 im Bereich der Nackenkontur 27 des Haaransatzes miteinander ein gleichschenkliges Kreuz 22 gemäß Fig. 9 bilden. Beispielsweise sind bei dieser zu behandelnden Person die Haarsträhnen auf Lockenwicklern bereits aufgewickelt und entsprechend mit Dauerwellflüssigkeit angefeuchtet. Zur Beschleunigung des Umwandlungsprozesses beim Haarkeratin wird jetzt das vorbeschriebene Dauerwellprogramm gestartet. Sollte aufgrund der besonderen Haarstruktur, beispielsweise bei dickem Haar die vom Dauerwellprogramm vorgegebene Einwirkzeit nicht ausreichen, so wird durch Betätigung der mit "N" bezeichneten Drucktaste 84 (Fig. 10) diese Einwirkzeit um einen vorgegebenen Wert entsprechend verlängert. Durch ein optische umd am Ende auch akustisches Warnsignal wir die Bedienungsperson des Wärmebehandlungsgerätes auf das Ende der Wärmebehandlung hingewiesen. Wahlweise kann auch entsprechend eines der anderen zwei Programme in der vorbeschriebenen Art gestartet werden.

In Fig. 11 wird eine zweite Ausführungsform des erfindungsgemäßen Wärmebehandlungsgerätes gezeigt, bei dem abweichend zur bisher in Fig. 1 bis 10 beschriebenen Ausführungsform statt eines Stativkreuzes 8 ein Wamdarm 42 den Tragearm 7 trägt. Dieser Wandarm 42 besteht in bekannter Weise sus einem an der Wand befestigtem Wandsockel 43 auf dem ein um eine vertikale Achse 44 drehbarer Oberarm 45 befestigt ist, an dessem anderen Ende ein um eine vertikale Achse 46 drehbarer und um eine horizontale Achse 47 vertikal wahlweise schwenkbarer Unterarm 49 angeordnet ist. Am anderen Ende des Unterarms 49 ist über eine Parallelführung ein Fortsatz 51 um die horizontale Achse 50 angelenkt, der den wahlwetse horizontal verdrehbaren Tragearm 7 trägt. In bekannter Weise können somit die Infrarotstrahler 6 und 9 mittels des Griffes lOa in jede beliebige Stellung relativ zum behandelnden Kopf 14 bei vorgegebenem Mindestabstand D eingerichtet werden.

Bei einer weiteren nicht dargestellten Ausführungsform können statt der zwei Projektionslampen 17 tragenden optischen Einstellvorrichtung andere Abstandsvorrichtungen, z.B. mechanischer Art, zur Einrichtung des Mindestabstand des Kopfes 14 von den Infrarotstrahlern 6 und 9 verwendet werden.

**Patentansprüche**

1 Wärmebehandlungsgerät zum Erwärmen der menschlichen Kopfhaare mit mehreren an mindestens einem Tragearm (7) befestigten als Flächenstrahler ausgebildeten Infrarotstrahlern (6, 9), dadurch gekennzeichnet, daß die Infrarotstrahler (6, 9) im wesentlichen als Kreissegment gekrümmte linienförmige Heizkörper (23) ausgebildet sind und jeweils einen außenkreissigen, im vesentlichen koaxial zum Heiskörper (23) gekrümmten,metallischen Reflektor (24) aufweisen, daß zwei seitliche Infrarotstrahler (6) und wenigstens ein in Gebrauchslage oberhalb angeordneter mittlerer Infrarotstrahler (9) mit mindestens einem an einem Bodenstativ (2, 3) bzw. an einem Wandarm (42) beweglich gelagerten Tragearm (7) fest verbunden sind, daß die in Richtung eines Krümmungsradius (R) jeweils verlaufenden Mittelachsen (26) zweier seitlich angeordneter Infrarotstrahler (6) miteinander einen Winkel (β) von wenigstens 80° einschließen und daß das Verhältnis aus Länge (L) des gekrümmten linienförmigen Heizkörpers (23) zu dessen Krümmungsradius (R) wenigstens 0,2 beträgt.

2. Wärmebehandlungsgerät nach Anspruch 1, dadurch gekennzeichnet, daß zwei mittlere Infrarotstrahler (9) angeordnet sind, deren in Richtung eines Krümmungsradius (R) verlaufende Mittelachsen (28) miteinander einen Winkel (γ) von wenigstens 45°, vorzugsweise von etwa 68,5° einschließen und daß vorzugsweise die Mittelachse (28) des in Gebrauchslage tiefer angeordneten mittleren Infrarotstrahlers (9) in dieser Gebrauchslage einen Erhebungswinkel (ψ) von wenigstens 30°, vorzugsweise von 55° mit der Horizontalen (29) bildet (Fig. 6).

3. Wärmebehandlungsgerät nach Anspruch 1, dadurch gekennzeichnet, daß die in Richtung eines Krümmungsradius (R) verlaufenden Mittelachsen (26) der seitlichen Infrarotstrahler (6) miteinander einen Winkel (β) von wenigstens 100°, vorzugsweise von 108° einschließen und daß vorzugsweise die Längsachse jedes der seitlichen Infrarotstrahler (6) in Gebrauchslage einen Erhebungswinkel (φ) von wenigstens 5, vorzugsweise 10 bis 12 mit der Horizontalen (29) bildet (Fig. 1 und 5).

4. Wärmebehandlungsgerät nach Anspruch 1 und/oder 3, dadurch gekennzeichnet, daß die Mittelachsen (26) der seitlichen Infrarotstrahler (6) in Gebrauchslage nach oben geneigt sind und mit der Horizontalen (29) einen Winkel (ε) von vorzugsweise 10° bilden (Fig. 7).

5. Wärmebehandlungsgerät nach Anspruch 1 und 4, dadurch gekennzeichnet, daß die seitlichen Infrarotstrahler (6) von einer ersten Gebrauchslage um einen Winkel (ε + λ) von mindestens 15°, vorzugsweise 30° nach unten in eine zweite vorzugsweise gerastete Gebrauchslage geschwenkt werden können (Fig. 8).

6. Wärmebehandlungsgerät nach Anspruch 1, dadurch gekennzeichnet, daß die seitlichen Infrarotstrahler (6) mittels eines zum Tragearm (7) benachbart liegenden Gelenkes (19) um eine im wesentlichen senkrechte Achse von der Gebrauchsstellung in die Mitte aufeinander zu in eine Ruhestellung geklappt werden können, so daß die seitlichen Infrarotstrahler (6) unterhalb des mittleren Infrarotstrahlers (9) zu liegen kommen (Fig. 4).

7. Wärmebehandlungsgerät nach Anspruch 1, gekennzeichnet durch eine Einstellvorrichtung zur optischen Lagefixierung, wobei vorzugsweise das Bild eines von zwei Projektionslampen (17) erzeugten Fadenkreuzes (20) im vorgegebenen Abstand an einer vorgegebenen Stelle, vorzugsweise im Sereich des Haaransatzes am Hals, nur scharf abgebildet wird (Fig. 1 und 5).

8. Wärmebehandlungsgerät nach Anspruch 1, dadurch gekennzeichnet, daß der linienförmige Heizkörper als Rohrheizkörper (23) aus Quarzglas ausgebildet ist und einen Krümmungsradius (R) von wenigstens 600 mm und höchstens von 1000 mm, vorzugsweise von 800 mm,aufweist (Fig. 5).

9. Wärmebehandlungsgerät nach Anspruch 1, dadurch gekennzeichnet, daß der Abstand (S bzw. H) vom gemeinsamen Schnittpunkt (M bzw. P) der Hauptstrahlrichtung (26 bzw. 28) der seitlichen Infrarotstrahler (6) bzw. des mittleren Infrarotstrahlers (9) von deren jeweiligem linienförmigem Heizkörper (23) wenigstens 30 %, vorzugsweise 50 % und maximal 70 % des mittleren Krümmungsradius (R) gemessen in Hauptstrahlrichtung beträgt (Fig. 5 bzw. 6).

10. Wärmebehandlungsgerät nach Anspruch 1, dadurch gekennzeichnet, daß die Länge (L) des linienförmigen Heizkörpers (23) der seitlichen Infrarotstrahler (6) bzw. des mittleren Infrarotstrahlers (9) wenigstens 30 %, vorzugsweise 37 bis 57 % seines mittleren Krümmungsradius (R) gemessen in Hauptstrahlrichtung beträgt (Fig. 5).

11. Wärmebehandlungsgerät nach Anspruch 1, dadurch gekennzeichnet, daß im Haar, vorzugsweise auf der Oberfläche eines mit einer Haarsträhne bewickelten Lockenwicklers (15), ein Temperaturfühler (12) angeordnet ist, der die Heizleistung der Infrarotstrahler (6,9) mittels einer vorzugsweise im Sedienpult (11) angeordneten Regelbausteins auf eine vorgegebene Solltemperatur einregelt (Fig. 1).

12. Wärmebehandlungsgerät nach Anspruch 1 und 11, dadurch gekennzeichnet, daß der Temperaturfühler (12) als ein in einem Kunststoffgehäuse gekapselt untergebrachter NTC-Widerstand ausgebildet ist und daß Befestigungsmittel an seinem Gehäuse vorgesehen sind, die diesen in unmittelbarem Kontakt mit dem auf dem Lockenwickler (15) aufgewickeltem Haar halten.

13. Wärmebehandlungsgerät nach Anspruch 1 und/oder 12, dadurch gekennzeichnet, daß ein die notwendigen elektrischen Steuer- und Regelbausteine enthaltendes Bedienpult (11) vorgesehen ist, das mittels eines Mikroprozessors die Steuerung der Heizleistung des Heizkörpers (23), vorzugsweise mitlels Triac-Halbleiter über das Impuls-Pausenverhältnis des Heizstromes, steuert und vorzugsweise auf Einhaltung einer solltemperatur am Temperaturfühler (12) hin regelt.

14. Wärmebehandlungsgerät nach Anspruch 1 und/oder 12, dadurch gekennzeichnet, daß die vom Infrarotstrahler (9 bzw. 6) erzeugte Infrarotstrahlung eine Wellenlänge von 2,3 bis 2,7μm, vorzugsweise von 2,5 μm aufweist.

15. Wärmebehandlungsgerät nach Anspruch 1 und 2, dadurch gekennzeichnet, daß die mittlere bzw. seitliche Infrarotstrahler (9,6) jeweils einen derart ausgebildeten metallischen Reflektor (24) aufweisen, der im Querschnitt ein gerades Mittelstück sowie zwei sich daran anschließende, sich V-förmig in Hauptstrahlungsrichtung um einen Öffnungswinkel (2δ) von vorzugsweise 60° erweiternde gerade Seitenanteile aufweist, die vorzugsweise symmetrisch zur Hauptstrahlrichtung (26) angeordnet sind (Fig. 7).

**Claims**

1. A heat treatment apparatus for heating human hair on the head, comprising a plurality of infra-red radiators (6, 9) secured to at least one support arm (7) and constructed in the form of surface radiators, characterized in that the infra-red radiators (6, 9) are constructed in the form of linear heating elements (23) curved substantially like a segment of a circle and comprise in each case a metal reflector (24) which is disposed on the outer periphery and which is bent substantially coaxially with the heater element (23), two lateral infra-red radiators (6) and at least one central infra-red radiator (9) disposed above in the position of use are rigidly connected to at least one support arm (7) displaceably mounted on a ground stand (2, 3) or a wall bracket (42), the median axes (26) - extending in each case in the direction of a radius of curvature (R) - of two laterally disposed infra-red radiators (6), form between them an angle (β) of at least 80°, and the ratio of the length (L) of the curved linear heater element (23) to the radius of curvature (R) thereof is at least 0.2.

2. A heat treatment apparatus according to Claim 1, characterized in that two central infra-red radiators (9) are provided, whose median axes (28) extending in the direction of a radius of curvature (R) form between them an angle (γ) of at least 45°, preferably approximately 68.5°, and the median axis (28) of the central infra-red radiator (9) disposed at a lower location in the position of use preferably forms in this position of use an angle of elevation (ψ) of at least 30°, preferably 55°, with the horizontal (29) (Fig. 6).

3. A heat treatment apparatus according to Claim 1, characterized in that the median axes (26)-extending in the direction of a radius of curvature (R) - of the lateral infra-red radiators (6) form between them an angle (β) of at least 100°, preferably 108°, and the longitudinal axis of each of the lateral infra-red radiators (6) forms in the position of use an angle of elevation (φ) of at least 5°, preferably from 10 to 12°, with the horizontal (29) (Figs. 1 and 5).

4. A heat treatment apparatus according to Claim 1 and/or 3. characterized in that the median axes (26) of the lateral infra-red radiators (6) are inclined upwards in the position of use and form with the horizontal (29) an angle (ε) of preferably 10° (Fig. 7).

5. A heat treatment apparatus according to Claims 1 and 4, characterized in that the lateral infra-red radiators (6) can be swung downwards through an angle (ε + λ) of at least 15°, preferably 30°, from a first position of use into a second, preferably retained, position of use (Fig. 8).

6. A heat treatment apparatus according to Claim 1, characterized in that the lateral infra-red radiators (6) can be swung by means of a joint (19) adjacent the support arm (7) about a substantially vertical axis from the position of use into the centre towards one another into a rest position, so that the lateral infra-red radiators (6) come to rest below the central infra-red radiator (9) (Fig. 4).

7. A heat treatment apparatus according to Claim 1, characterized by a setting device for setting the optical position, the image of a crosswire (20) produced by two projector lamps (17) preferably being only sharply reproduced at the predetermined distance at a predetermined location, preferably in the region of the hair line on the neck (Figs. 1 and 5).

8. A heat treatment apparatus according to Claim 1, characterized in that the linear heating element is constructed as a tubular heater element (23) of quartz glass and has a radius of curvature (R) of at least 600 mm and at most 1000 mm, preferably 800 mm (Fig. 5).

9. A heat treatment apparatus according to Claim 1, characterized in that the distance (S and H respectively) from the common intersection point (M and P respectively) of the main radiation direction (26 and 28 respectively) of the lateral infra-red radiators (6) or of the central infra-red radiator (9) from the respective linear heater element (23) thereof respectively amounts to at least 30%, preferably 50% and at most 70% of the mean radius of curvature (R) measured in the main radiation direction (Figs. 5 and 6 respectively).

10. A heat treatment apparatus according to Claim 1, characterized in that the length (L) of the linear heater element (23) of the lateral infra-red radiators (6) or of the central infra-red radiator (9) respectively amounts to at least 30%, preferably 37 to 57% of its mean radius of curvature (R) measured in the main radiation direction (Fig. 5).

11. A heat treatment apparatus according to Claim 1, characterized in that a temperature sensor (12). which sets the heating power of the infra-red radiators (6. 9) to a predetermined nominal temperature by means of a control module preferably arranged in the control panel (11), is arranged in the hair, preferably on the surface of a curler (15) around which is wound a strand of hair (Fig. 1).

12. A heat treatment apparatus according to Claims 1 and 11, characterized in that the temperature sensor (12) is constructed in the form of an NTC resistor embedded in a plastics casing, and securing means are provided on its casing which hold it in direct contact with the hair wound around the curler (15).

13. A heat treatment apparatus according to Claim 1 and/or 12, characterized in that a control panel (11) is provided, which contains the necessary electrical control and regulator modules and controls by means of a microprocessor the control of the heating power of the heater element (23), preferably by means of triac semi-conductors by way of the pulse-pause ratio of the heating current and preferably regulates it so as to maintain a nominal temperature at the temperature sensor (12).

14. A heat treatment apparatus according to Claim 1 and/or 12, characterized in that the infra-red radiation produced by the infra-red radiators (9 and 6 respectively) has a wavelength of from 2.3 to 2.7 μm, preferably 2.5 μm.

15. A heat treatment apparatus according to Claims 1 and 2, characterized in that the central and lateral infra-red radiators (9, 6) respectively, each comprise a metal reflector (24) of the type which comprises in cross-section a straight centre member as well as two straight side members which are adjacent thereto and which widen in a V-shaped manner in the main radiation direction by an opening angle (2δ) of preferably 60° and which are preferably arranged symmetrically to the main radiation direction (26) (Fig. 7).

**Revendications**

1. Appareil de traitement par la chaleur pour chauffer les cheveux d'une personne, comprenant plusieurs émetteurs de rayons infrarouges (6, 9) fixés sur au moins un bras porteur (7) et constitués sous forme d'émetteurs en nappe, caractérisé en ce que les émetteurs de rayons infrarouges (6, 9) sont constitués essentiellement sous forme de corps chauffants (23) de forme linéaire et courbe sensiblement en segment de cercle et possèdent chacun un réflecteur métallique (24) courbé sensiblement coaxialement au corps chauffant (23) sur le côté extérieur de la courbure, que deux émetteurs de rayons infrarouges latéraux (6) et au moins un émetteur de rayons infrarouges central (9) disposé au-dessus en position d'utilisation sont fermement fixés à au moins un bras porteur (7) monté de façon mobile sur un pied (2, 3) ou sur un bras mural (42), que les axes centraux (26) respectivement orientés dans la direction d'un rayon de courbure (R) de deux emetteurs de rayons infrarouges (6) disposés latéralement forment entre eux un angle ( β) d'au moins 80° et que le rapport entre la longueur (L) du corps chauffant de forme linéaire et courbe (23) et son rayon de courbure (R) est d'au moins 0,2.

2. Appareil de traitement par la chaleur selon la revendication 1, caractérisé en ce que sont prévus deux émetteurs de rayons infrarouges centraux (9), dont les axes centraux (28) orientés en direction d'un rayon de courbure (R) forment ensemble un angle (γ) d'au moins 45°, et de préférence d'environ 68,5°, et de préférence que l'axe central (28) de l'émetteur de rayons infrarouges central (9) disposé le plus en bas en position d'utilisation forme dans cette position d'utilisation un angle d'élévation (ψ) d'au moins 30°, et de préférence de 55° avec l'horizontale (29) (figure 6).

3. Appareil de traitement par la chaleur selon la revendication 1, caractérisé en ce que les axes centraux (26) orientés en direction d'unrayon de courbure (R) des émetteurs de rayons infrarouges latéraux (6) forment ensemble un angle (β) d'au moins 100°, et de préférence de 108°, et de préférence que l'axe longitudinal de chaque émetteur de rayons infrarouges latéral (6) forme en position d'utilisation un angle d'élévation (γ ) d'au moins 5°, et de préférence de 10 à 12° avec l'horizontale (29) (figures 1 et 5).

4. Appareil de traitement par la chaleur selon la revendication 1 et/ou 3, caractérisé en ce que les axes centraux (26) des émetteurs de rayons infrarouges latéraux (6) sont inclinés vers le haut en position d'utilisation et forment avec l'horizontale (29) un angle (ε ) de preférence de 10° (figure 7).

5. Appareil de traitement par la chaleur selon les revendications 1 et 4, caractérisé en ce que les émetteurs de rayons infrarouges latéraux (6) peuvent être pivotés à partir d'une première position d'utilisation sur un angle (ε + λ) d'au moins 15° et de préférence de 30° vers le bas vers une seconde position d'utilisation, de préférence verrouillée (figure 8).

6. Appareil de traitement par la chaleur selon la revendication 1, caractérisé en ce que les émetteurs de rayons infrarouges latéraux (6) peuvent être rabattus l'un vers l'autre au centre depuis la position d'utilisation vers une position de repos au moyen d'une articulation (19) située au voisinage du bras porteur (7) et autour d'un axe sensiblement vertical, de manière que les émetteurs de rayons infrarouges latéraux (6) puissent se disposer au-dessous des émetteurs de rayons infrarouges centraux (9) (figure 4).

7. Appareil de traitement par la chaleur selon la revendication 1, caractérisé par un dispositif de réglage pour déterminer optiquement la position, l'image d'un réticule (20) formé par deux projecteurs (17) étant tracée de façon précise à une distance prédéterminée et dans une position prédéterminée, de préférence dans la région de la naissance des cheveux sur le cou (figures 1 et 5).

8. Appareil de traitement par la chaleur selon la revendication 1, caractérisé en ce que le corps chauffant de forme linéaire est constitué sous forme d'un corps tubulaire chauffant en verre quartzeux et présente un rayon de courbure (R) d'au moins 600 mm, au maximum de-1000 mm et de préférence de 800 mm (figure 5).

9. Appareil de traitement par la chaleur selon la revendication 1, caractérisé en ce que la distance (S ou H) entre le point d'intersection commun (M ou P) de la direction du rayonnement principal (26 ou 28) des émetteurs de rayons infrarouges latéraux (6) ou des émetteurs de rayons infrarouges centraux (9) des corps chauffants de forme linéaire respectifs (23) est d'au moins 30%, de préférence 50% et au maximum de 70% du rayon de courbure moyen (R) mesuré dans la direction principale du rayonnement (figures 5 et 6).

10. Appareil de traitement par la chaleur selon la revendication 1, caractérisé en ce que la longeur (L) du corps chauffant de forme linéaire (23) des émetteurs de rayons infrarouges latéraux (6) ou de l'émetteur de rayons infrarouges central (9) est d'au moins 30%, de preférence entre 37 et 50%, de son rayon de courbure moyen (R) mesuré dans la direction principale du rayonnement (figure 5).

11. Appareil de traitement par la chaleur selon la revendication 1, caractérisé en ce que dans les cheveux, et de préférence sur la surface d'un bigoudi (15) sur lequel est enroulé une mèche de cheveux, est disposé un détecteur de température (12), qui régule la puissance de chauffage des émetteurs de rayons infrarouges (6, 9) sur une température de consigne prédéterminée au moyen d'un bloc de régulation monté de préférence dans le pupitre de service (11) (figure 1).

12. Appareil de traitement par la chaleur selon les revendications 1 et 11, caractérisé en ce que le détecteur de température (12) est constitué sous forme d'une résistance NTC encapsulée dans une enveloppe en matière plastique et que le moyen de fixation est prévu sur son enveloppe, qui maintient cette dernière en contact direct avec les cheveux enroulés sur le bigoudi (15).

13. Appareil de traitement par la chaleur selon la revendication 1 et/ou 12, caractérisé en ce qu'il est prévu un pupitre de service (11) contenant le bloc de commande et de régulation électrique nécessaire, qui contrôle au moyen d'un microprocesseur la commande de la puissance de chauffage du corps chauffant (23) de préférence au moyen d'un semiconducteur triac, par l'intermédiaire du rapport entre impulsions et pauses du courant de chauffage, et de préférence en maintenantune température de consigne pour le détecteur de température (12).

14. Appareil de traitement par la chaleur selon la revendication 1 et/ou 12, caractérisé en ce que le rayonnement infrarouge émis par l'émetteur de rayons infrarouges (9 ou 6) a une longueur d'onde comprise entre 2,3 et 2,7 μm, et de préférence de 2,5 μm.

15. Appareil de traitement par la chaleur selon la revendication 1 et 2, caractérisé en ce que les émetteurs de rayons infrarouges centraux ou latéraux (9, 6) comprennent chacun un réflecteur métallique (24) qui présente en section transversale un élément central rectiligne ainsi que deux éléments latéraux rectilignes qui s'y raccordent et vont en s'élargissant en forme de V en direction du rayonnement principal, selon un angle d'ouverture ($2\delta$) qui est de préférence de 60°, et qui sont de préférence disposés symétriquement par rapport à la direction principale (26) du rayonnement (figure 7).

Fig. 1

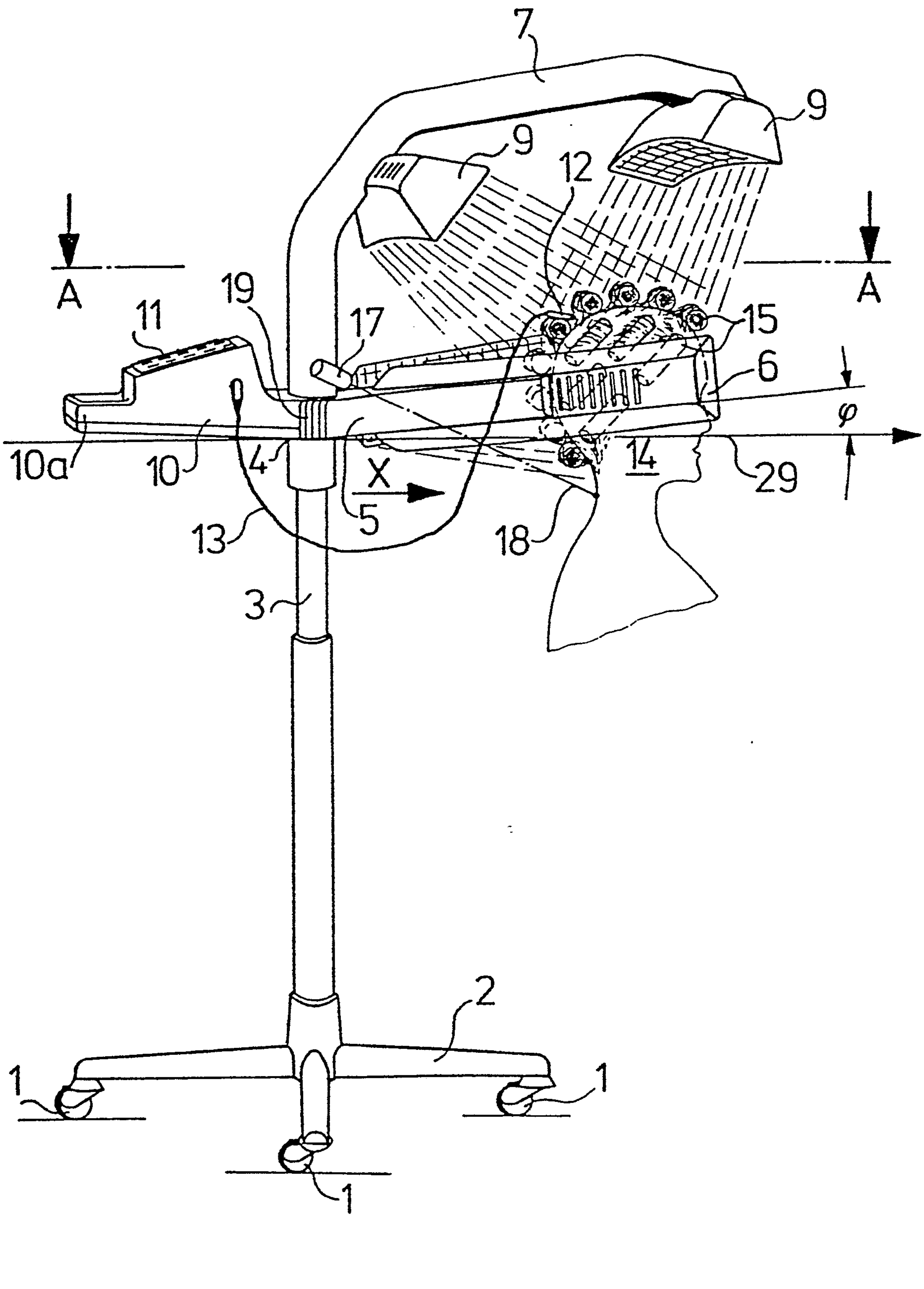

Fig. 2

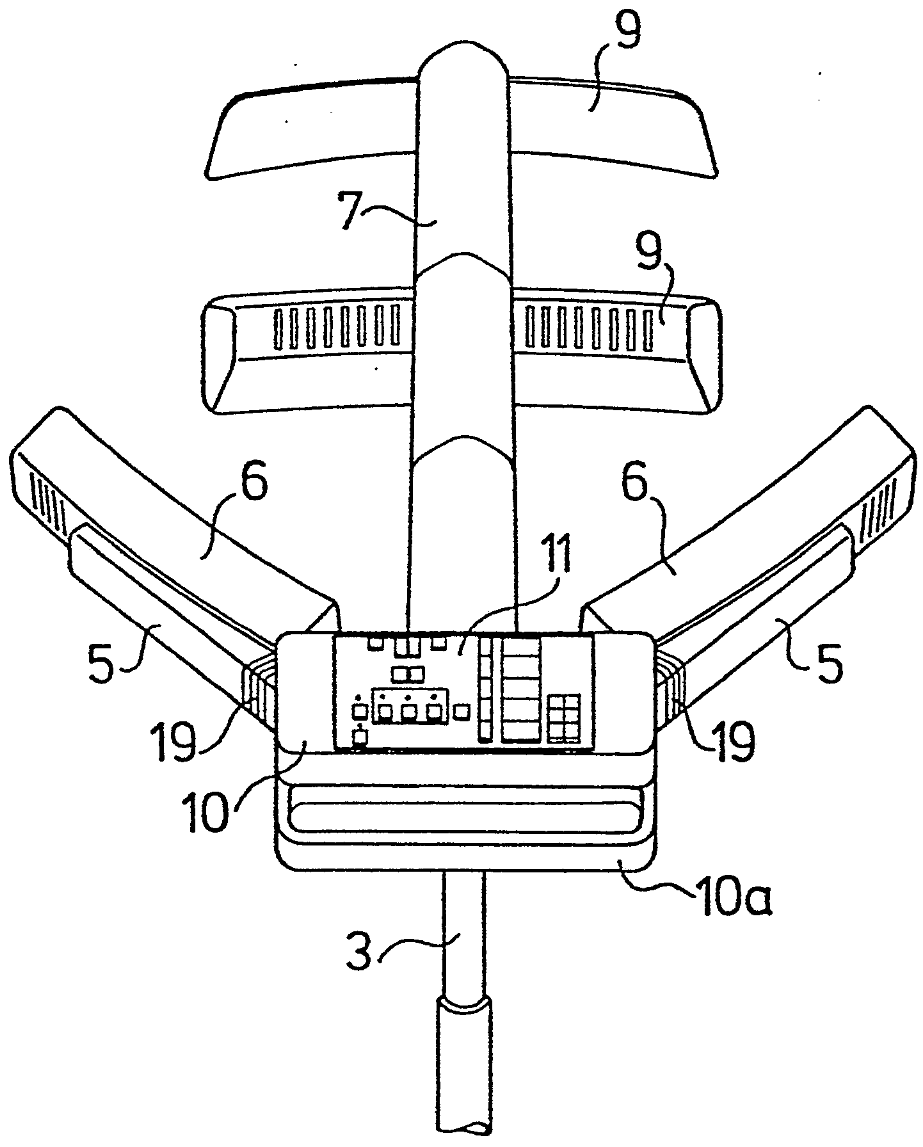

Fig. 3

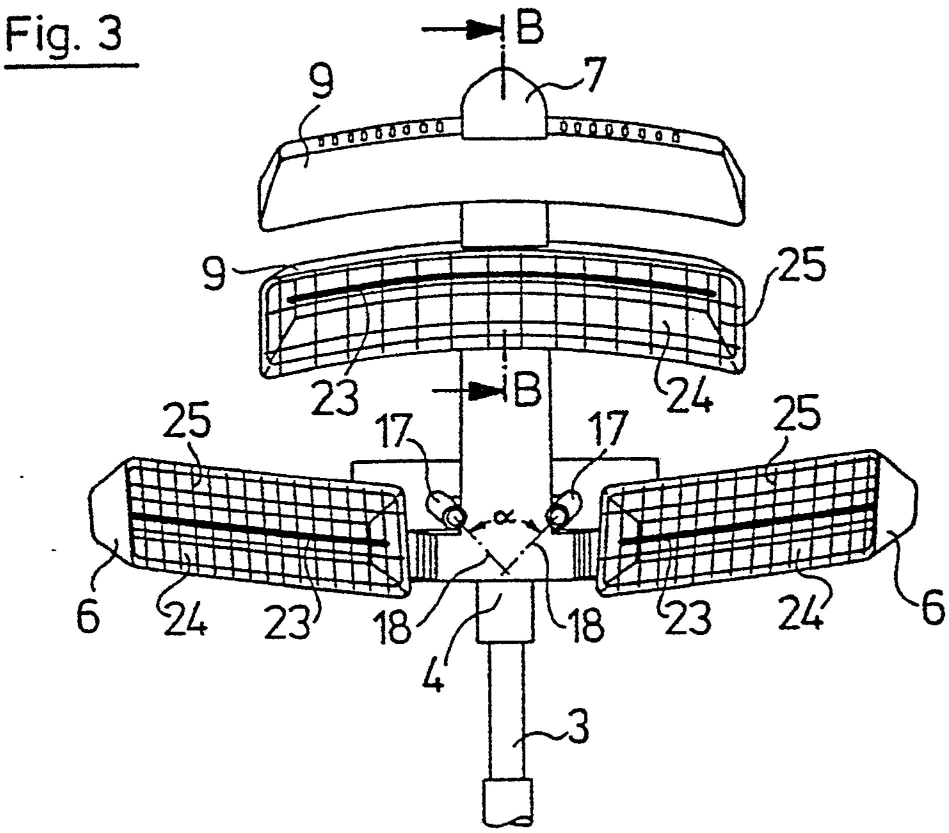
B
7
9
9
25
23
B
24
25
17
17
25
α
6
24
23
18
18
23
24
6
4
3

Fig. 4

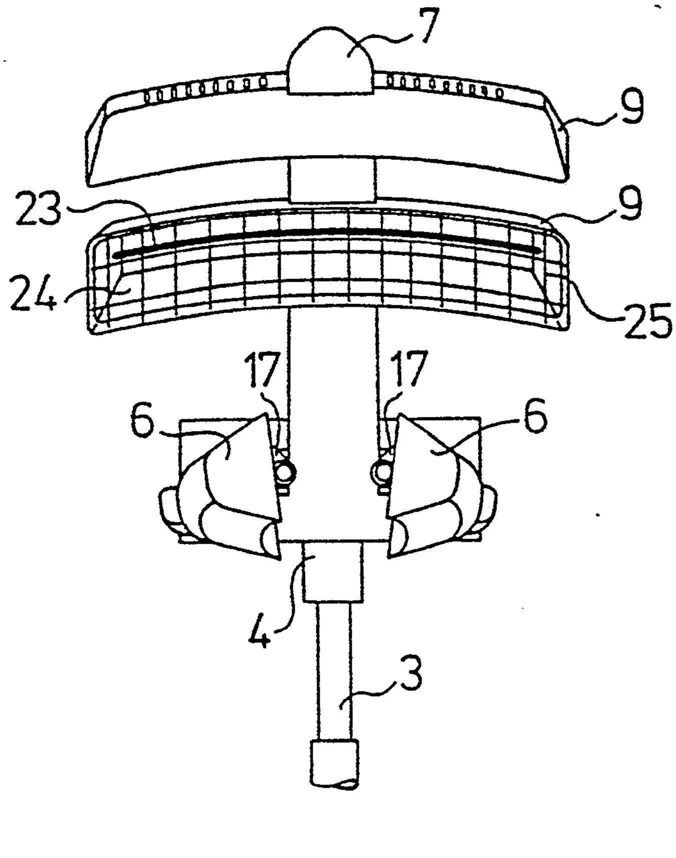
7
9
23
9
24
25
17
17
6
6
4
3

Fig. 5

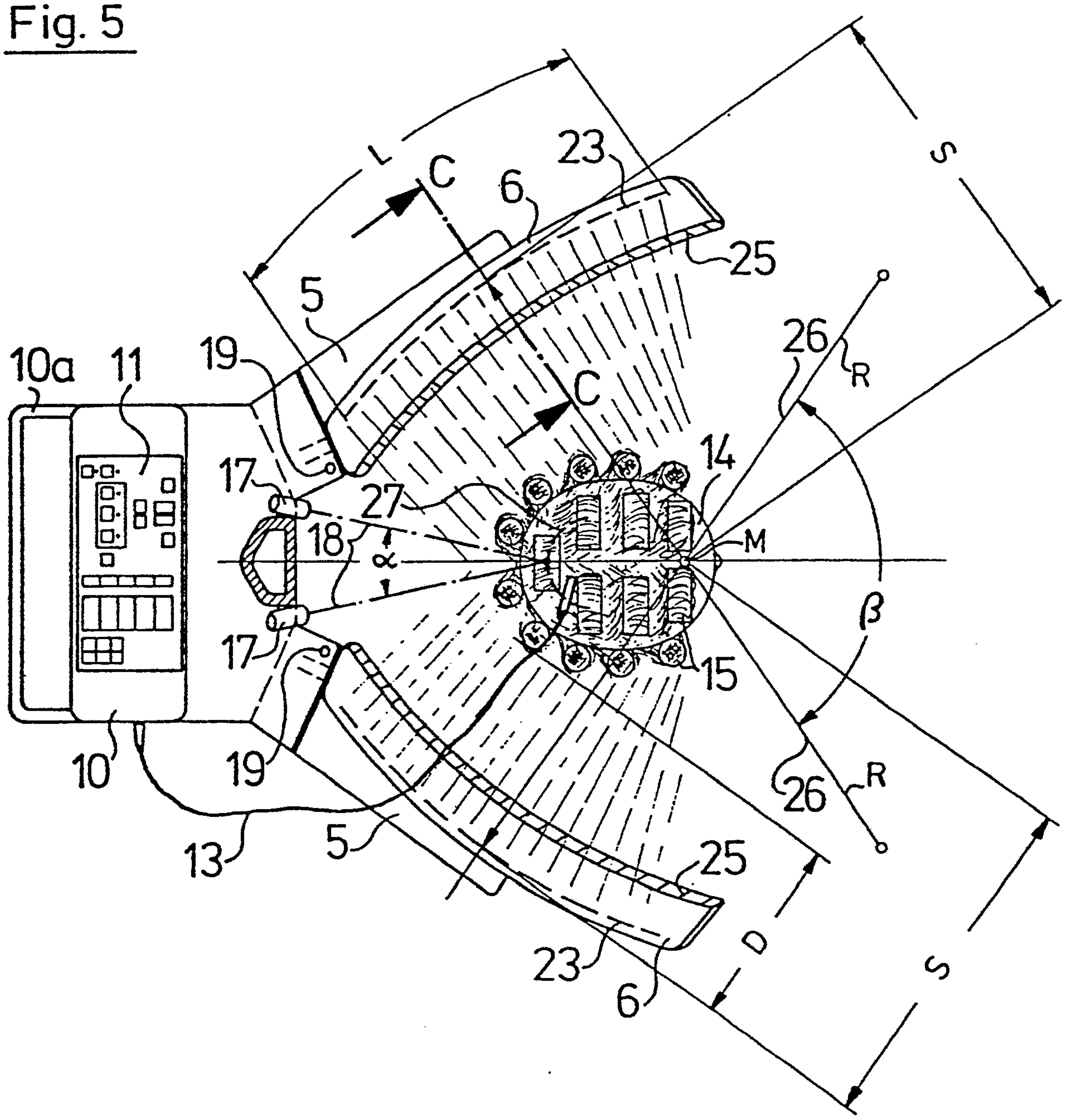
L
C
23
6
25
5
19
10a
11
17
27
18
α
14
M
26
R
β
15
17
19
10
13
5
26
R
25
23
6
D
S
S
C

Fig. 6

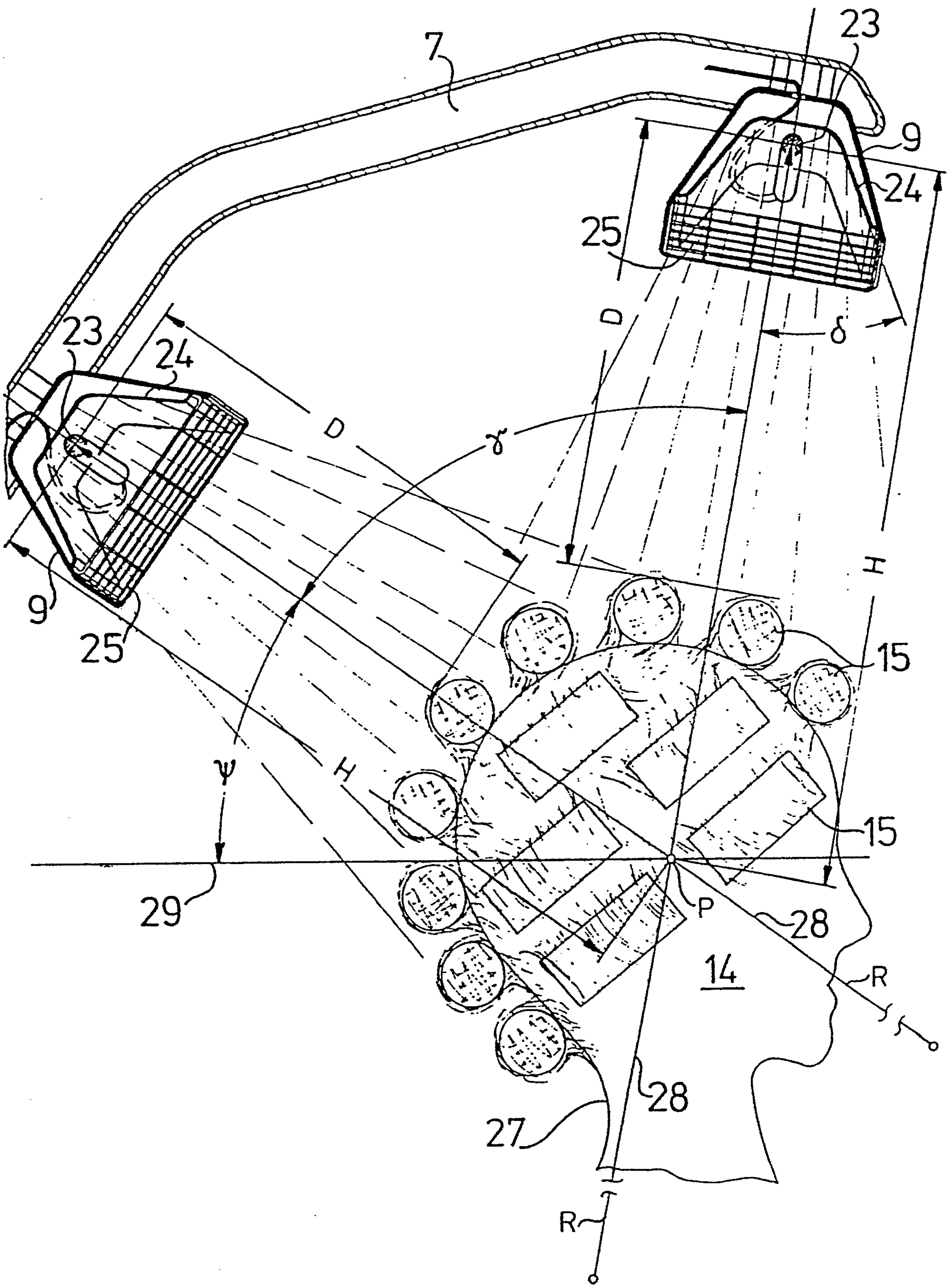

Fig. 7

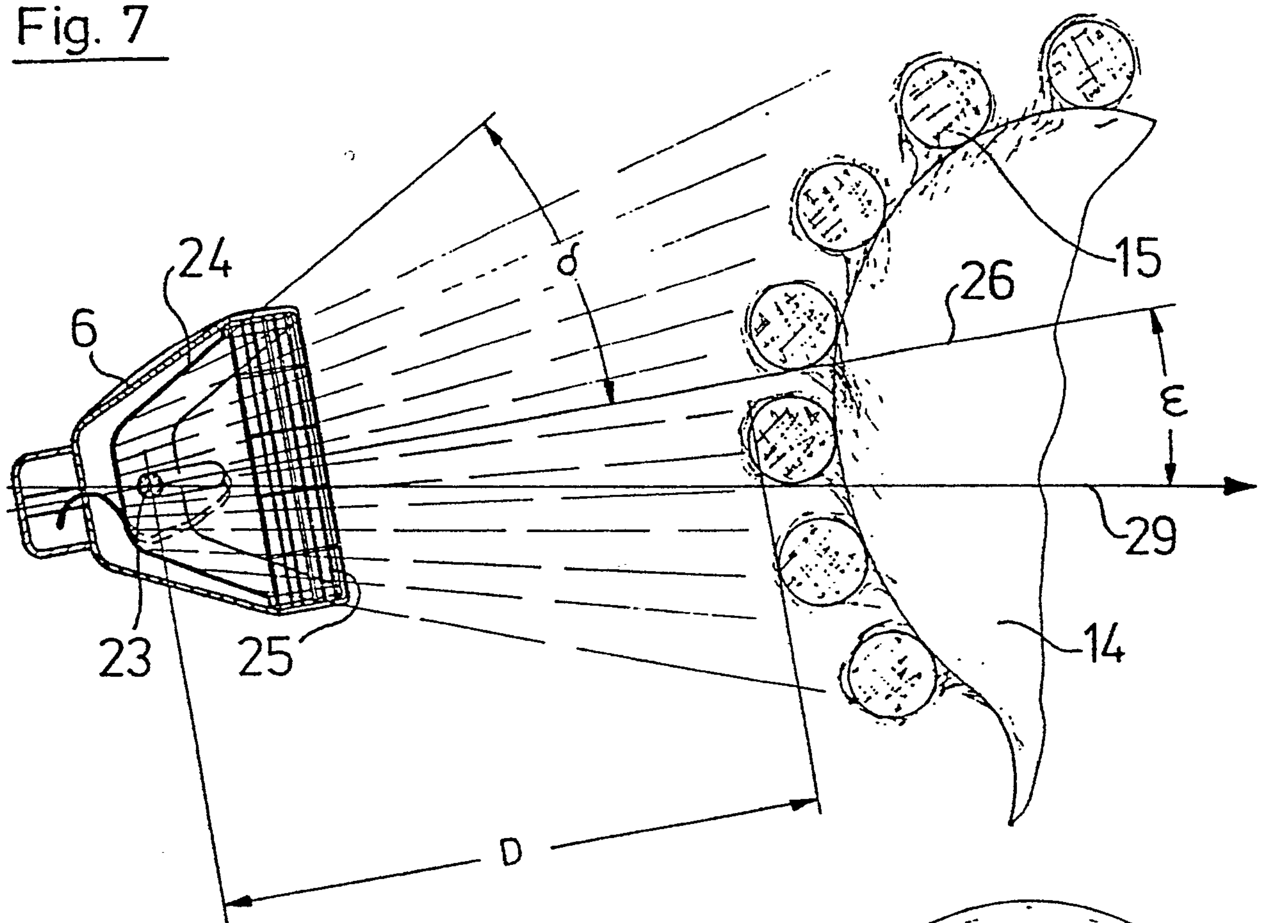


Fig. 8

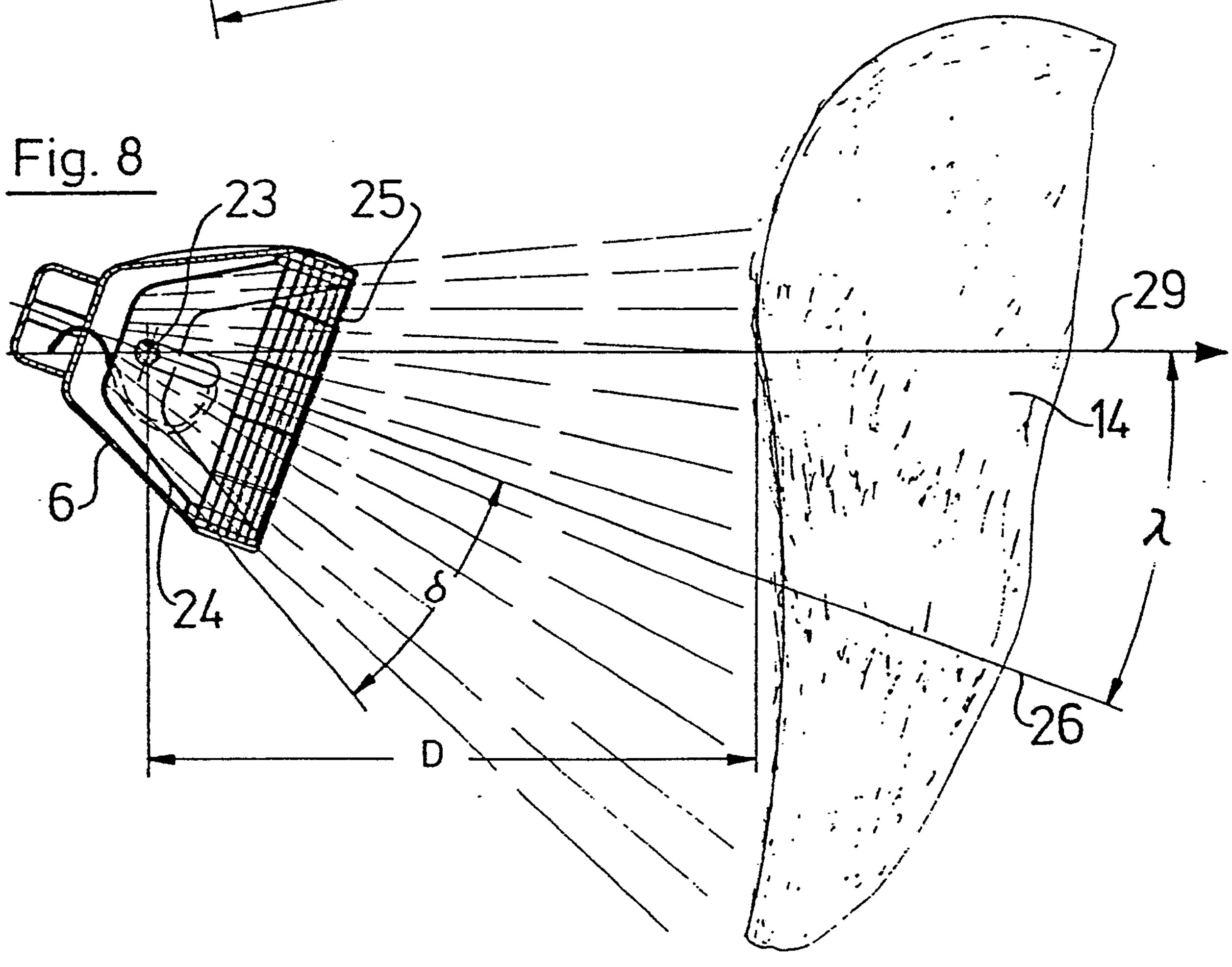

Fig. 9

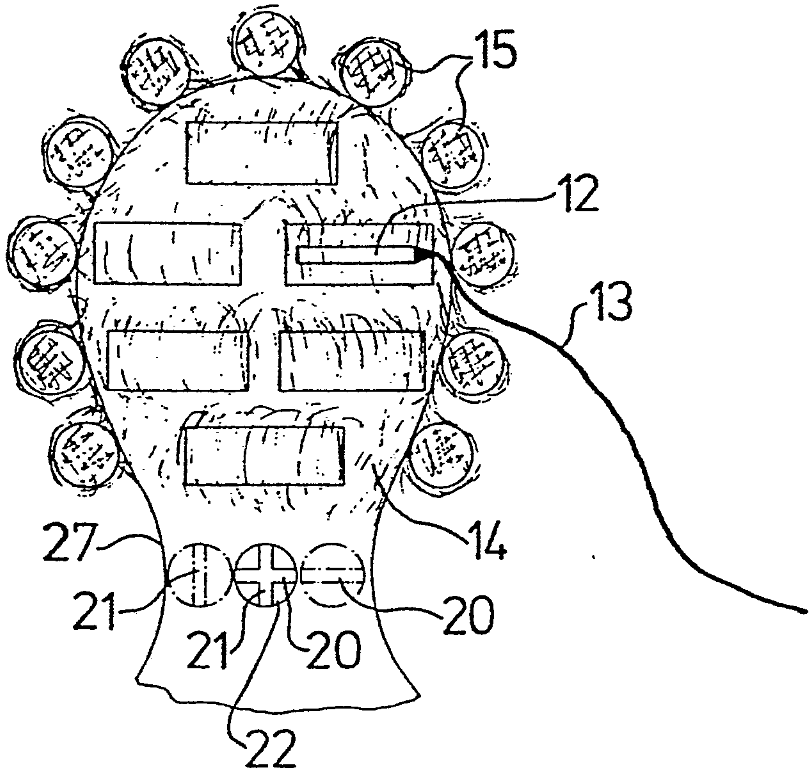
15
12
13
14
27
21
21
20
20
22

Fig. 10

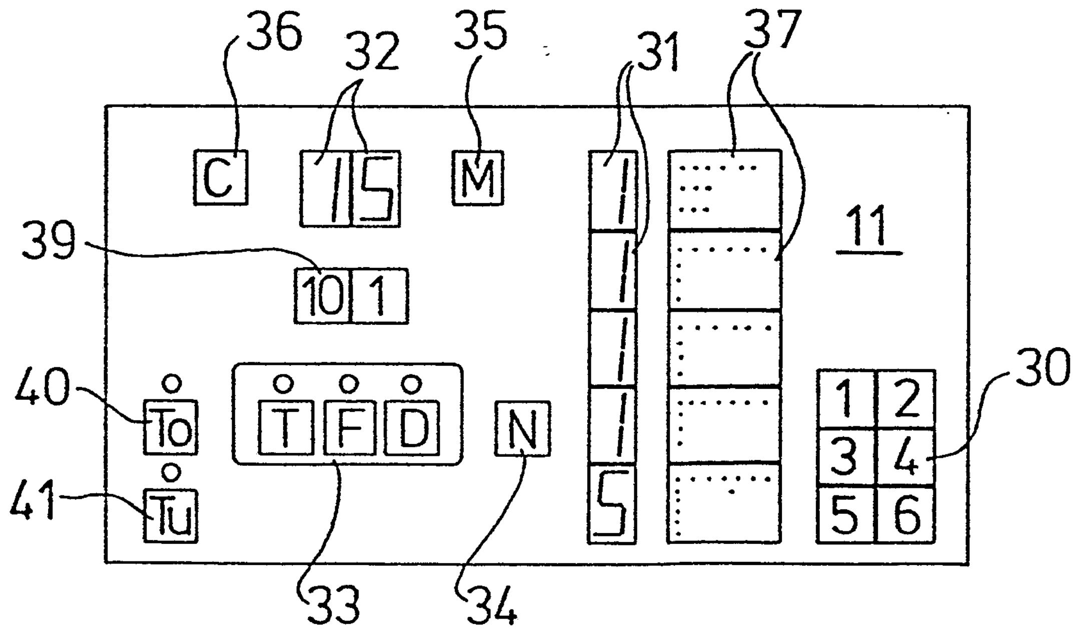
36
32
35
31
37
C
15
M
11
39
10
1
40
To
T
F
D
N
1
2
3
4
5
6
30
41
Tu
5
33
34

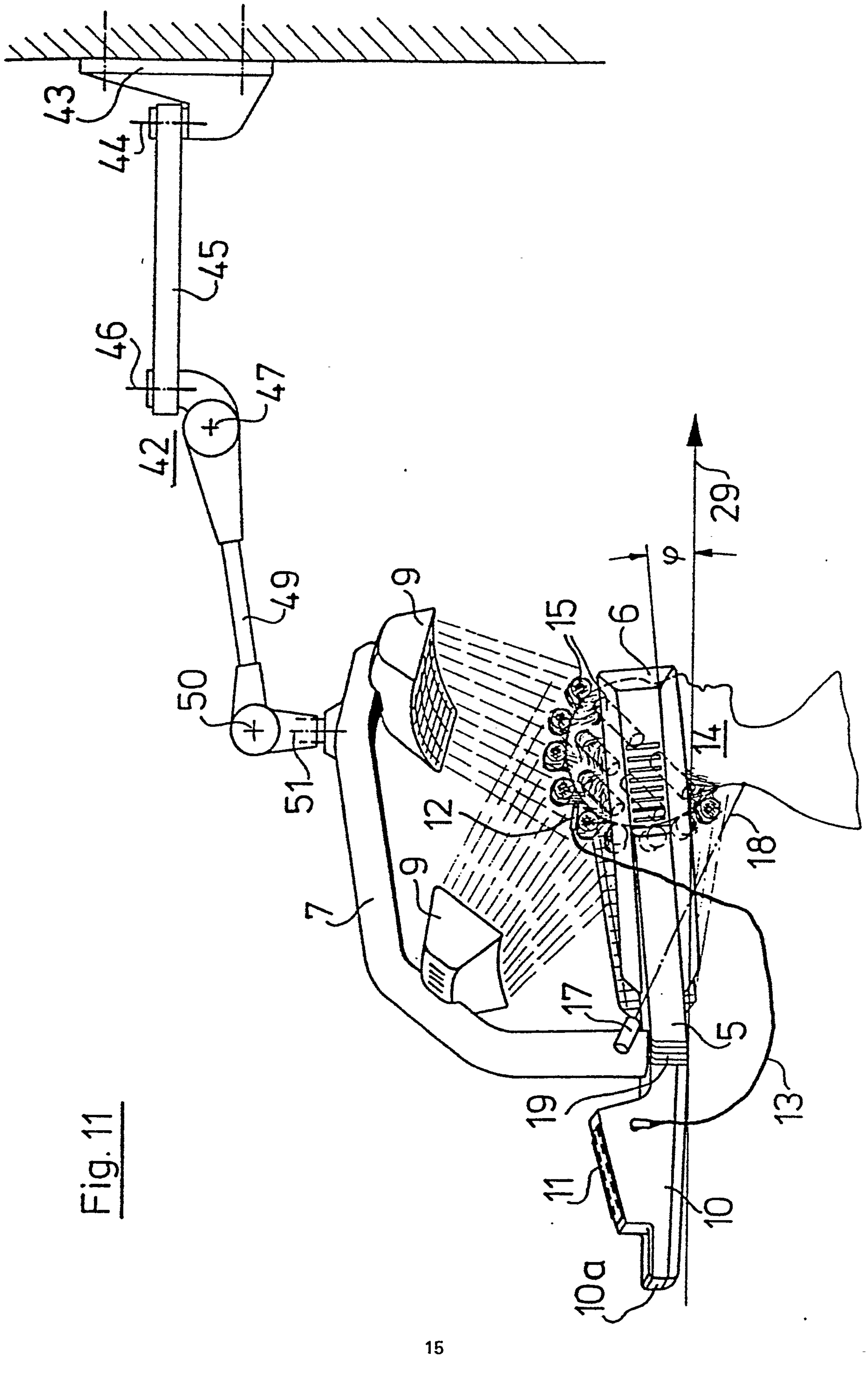


Fig. 11